# EUROPEAN PATENT SPECIFICATION

(11) **EP 2 313 453 B1**
(45) Date of publication and mention of the grant of the patent: **23.01.2013**
(21) Application number: 09790089.8
(22) Date of filing: 07.07.2009
(51) Int. Cl.: C08G 59/06

(54) **PRODUCTION OF SOLID EPOXY RESIN**
HERSTELLUNG VON EPOXIDFESTHARZ
PRODUCTION D'UNE RÉSINE ÉPOXY SOLIDE

(30) Priority: 05.08.2008 US 86311 P
(43) Date of publication of application: 27.04.2011
(73) Proprietor: Dow Global Technologies LLC, Midland, MI 48674 (US)
(72) Inventor: CARLBERG, Philip, Lake Jackson TX 77566 (US); GU, Leming, Lake Jackson TX 77566 (US); PHAM, Ha, Lake Jackson TX 77566 (US); RIPPLINGER, Eric, Lake Jackson TX 77566 (US); WEST, David, Houston TX 77041 (US); WORLEY, William, Missouri City TX 77459 (US); YOUNG, Thomas, Lake Jackson TX 77566 (US)
(74) Representative: Hull, John Philip
(86) International application number: PCT/US2009/049737
(87) International publication number: WO 2010/016987

(56) References cited:
- EP-A2- 0 340 716
- WO-A1-96/30433
- US-A- 4 518 762
- US-A- 6 001 873
- US-A- 6 063 876
- ANONYMOUS: "Co-solvents for making epoxy resins" RESEARCH DISCLOSURE, vol. 355, no. 028, 10 November 1993 (1993-11-10), XP002554464 Emsworth, GB
- DATABASE WPI Week 200125 Thomson Scientific, London, GB; AN 2001-239813 XP002554243 & JP 2001 040065 A (DAINIPPON INK & CHEM INC) 13 February 2001 (2001-02-13)
- DATABASE WPI Week 198248 Thomson Scientific, London, GB; AN 1982-03127J XP002554244 & JP 57 170922 A (TOHTO KASEI KK) 21 October 1982 (1982-10-21)
- DATABASE WPI Week 198431 Thomson Scientific, London, GB; AN 1984-191797 XP002554245 & JP 59 108072 A (NITTO ELECTRIC IND CO) 22 June 1984 (1984-06-22)

## Description

This application is a non-provisional application claiming priority from the U.S. Provisional Patent Application No. 61/086,311, filed on August 5, 2008, entitled "PRODUCTION OF SOLID EPOXY RESIN," the teachings of which are incorporated by reference herein, as if reproduced in full hereinbelow.

This invention relates generally to an improved method of producing glycidyl ether, more specifically a glycidyl derivative of a compound having at least one aromatic hydroxyl group or aromatic amine group per molecule. The glycidyl ether or glycidyl derivative may be more commonly referred to as an epoxy resin. This invention relates more particularly to preparation of solid epoxy resins (SERs) (as opposed to liquid epoxy resins (LERs)), especially those that have a molecular weight sufficient to be classified as medium to high molecular weight.

Production of SERs by reacting a polyhydric phenol, an epihalohydrin and an alkali metal hydroxide or alkaline earth hydroxide without use of an organic solvent leads to problems in controlling product quality in terms of, for example, one or more of epoxy equivalent weight, the average molecular weight, product softening point, melt viscosity, and reactivity. The SER product forms a highly viscous resin phase at the end of the reaction (e.g. common SERs with an epoxy equivalent weight of 800 g/equivalent have a viscosity of more than (>) 20,000 centistokes (cSt) (0.02 square meters per second (m²/sec) at a temperature of 120 degrees centigrade (°C)). Such a viscosity limits one's ability to reliably control side reactions and leads to difficulty in processing the reaction product. In addition, such a viscosity makes removal of residual ionic species, such as the alkali metal hydroxide, the alkaline earth metal hydroxide and any halides produced during preparation of the SER, very difficult, thereby leading to a SER product with an undesirably high residual ionic content (e.g. more than 50 parts by weight per million parts by weight of SER (ppm) ionic chloride content). A coating prepared from a SER with a residual ionic chloride content of more than 50 ppm leads to susceptibility to blistering and corrosion. High residual ionic content also catalyzes advancement, branching and other reactions in molten resins at high temperatures (e.g. at least (≥) 120°C), which leads, in turn, to high variability in product properties such as epoxy equivalent weight and average molecular weight.

United States Patent (USP) 4,499,255 to Wang et al. teaches preparation of LERs by reacting at least one compound having at least one aromatic hydroxyl group or aromatic amine group per molecule with an excess of at least one epihalohydrin in the presence of an alkali metal hydroxide. Wang et al. requires use of an organic solvent that codistills with water and the epihalohydrin at a boiling point below the boiling point of other reactants or reaction mixture components. Wang et al. also requires continuous removal of water by means of codistillation at a rate such that reaction mixture water content stays below six percent by weight, based on total reaction mixture weight.

USP 2,694,694 to Greenlee discloses preparation of high melting point (> 115°C and up to 150°C or higher), high molecular weight (e.g. an epoxide equivalent of 1484 (Example 2) or higher (2065 in Example 4)) glycidyl ethers. Preparation includes a molecular or molar ratio of chlorohydrin to bis-phenol > 1 to 1, but generally less than (<) 1.2 to 1, and use of an aqueous alkali such as caustic soda.

USP 2,767,157 to Masters focuses upon improvements in production of resins, more particularly high melting point high molecular weight resins, by reacting a dihydric phenol with epichlorohydrin in the presence of aqueous caustic alkali. Masters requires use of a small amount of an organic solvent that is insoluble or substantially insoluble in water, but is a solvent for epichlorohydrin and, at elevated temperatures, functions as a solvent for the resin at those temperatures. The solvent must also be free of reactive groups. Illustrative solvents include high-flash naphtha, xylene, mineral spirits, toluene, and high boiling ethers (e.g. di-n-butyl ether) and ketones (e.g. cyclohexanone) that do not contain reactive groups.

USP 2,848,435 to Griffin et al. discusses a process for preparing epoxy resins that includes use of an inert solvent for the resin and a molar ratio of epihalohydrin per phenolic hydroxyl equivalent of more than 0.5:1. The inert solvent is an aliphatic secondary monohydric alcohol that is otherwise devoid of any reactive groups.

In some embodiments, this invention is an improved process for preparing a glycidyl ether, which process comprises subjecting a reaction mixture, which reaction mixture comprises an aromatic hydroxyl-containing compound, an epihalohydrin, water, and an inorganic hydroxide that is at least one of alkali metal hydroxide or an alkaline earth metal hydroxide, to conditions sufficient to produce a glycidyl ether resin that is a solid at room temperature (nominally 25 degrees centigrade), the aromatic hydroxyl-containing compound and the epihalohydrin being present in a mole-equivalent ratio of hydroxyl moieties of the aromatic hydroxyl-containing compound to moles (one mole-equivalent) of epihalohydrin that falls within a range of from 0.5:1 to 1:1, and the epihalohydrin and alkali metal hydroxide or alkaline earth metal hydroxide being present in a molar ratio of the hydroxide to the epihalohydrin that falls within a range of from 0.2:1. to 2:1, wherein the improvement comprises adding a reaction solvent to the reaction mixture, the reaction solvent being at least one alcohol ether that comprises both an ether moiety and an alcohol moiety.

When ranges are stated herein, as in a range of from 2 to 10, both end points of the range (e.g. 2 and 10) and each numerical value, whether such value is a rational number or an irrational number, are included within the range unless otherwise specifically excluded.

Unless stated to the contrary, implicit from the context, or customary in the art, all parts and percents are based on weight.

In the improved process noted above, one subjects a reaction mixture that comprises an aromatic hydroxyl-containing compound, an epihalohydrin, an inorganic hydroxide that is at least one of alkali metal hydroxide or an alkaline earth metal hydroxide, and a reaction solvent to conditions sufficient to produce a glycidyl ether resin that is a solid at room temperature. The aromatic hydroxyl containing compound and epihalohydrin are present in the mole ratio indicated in claim 1. In addition, the epihalohydrin and alkali metal hydroxide or alkaline earth metal hydroxide being present in a molar ratio of hydroxide to epihalohydrin that falls within a range of from 0.2:1 to 2:1. The at least one alcohol ether comprises, consists essentially of, consists of, or contains both an ether moiety and an alcohol moiety.

In some embodiments, begin the process by forming a first mixture of the polyhydric phenol (which has a first or initial content of unreacted phenolic hydroxyl groups), the hydroxide, water and the alcohol ether and then continue the process by adding the epihalohydrin to the first mixture to form a second mixture. Allow components of the second mixture to react for a period of time sufficient to reduce or lower the initial content of unreacted phenolic hydroxyl (OH) groups to a second, suitably low amount (e.g. preferably < 20x10⁻⁵ gram mole (gmol) equivalents phenolic OH per gram of resin, more preferably < 6x10⁻⁵ gram mole (gmol) equivalents phenolic OH per gram of resin) and yield a combination of an aqueous brine mixture and a resin mixture. Separate the aqueous brine mixture from the resin mixture and wash the resin mixture with water to remove from the resin mixture at least a portion of residual salt(s) or other ionic species that may be present in the resin mixture. Recover the resin from the resin mixture by conventional procedures such as evaporation or vacuum stripping.

The foregoing process may be modified by one or more of several options. For example, include only a portion of the alkali metal hydroxide or alkaline earth metal hydroxide in the first mixture and add remaining alkali metal hydroxide or alkaline earth metal hydroxide in one or more aliquots or portions to the second mixture as components of the second mixture react with one another. In a second option, add the epihalohydrin either in a single aliquot or gradually over a period of time (preferably within a range of from 10 minutes to 3 hours, more preferably from 10 minutes to 2 hours addition time, and still more preferably from 10 minutes to less than or equal to 60 minutes addition time). If desired, neutralize the second mixture after reaching the suitably low amount or level of unreacted phenolic hydroxyl groups by adding to said second reaction mixture any of carbon dioxide, an inorganic acid or an organic acid. In a third option, start with a mixture of the polyhydric phenol, epihalohydrin and reaction solvent as the first mixture and then add the hydroxide (alkali metal or alkaline earth metal) to form the second mixture. Hydroxide addition may occur in a single aliquot or over a period of time such as any time within a range of from 10 minutes to 120 minutes. Skilled artisans understand that components of the second mixture react, at least to some extent, during addition of the hydroxide over time. In any part of the foregoing process, with or without one or more of the options discussed in this paragraph, a further option includes or comprises adding an amount of a dilution solvent to the first mixture, the second mixture, the combination of an aqueous brine mixture and a resin mixture, or the resin mixture itself before or during water washing of the resin mixture.

The dilution solvent is preferably substantially free of any moiety that reacts with one or more of the aromatic hydroxyl-containing compound, the epihalohydrin, the alkali metal hydroxide or alkaline earth metal hydroxide, and water. The dilution solvent also preferably has a boiling point at normal atmospheric pressure (14.7 pounds per square inch absolute (psia)) that is < 200°C. In addition, the dilution preferably forms an azeotrope with water, which azeotrope boils at a temperature less than a temperature at which water boils at normal atmospheric pressure.

As a variation of the foregoing process, include the epihalohydrin in the first mixture and add the alkali metal hydroxide or alkaline earth metal hydroxide to the first mixture to form the second mixture.

The improved process of this invention, which requires use of a solvent, specifically an alcohol ether solvent, with or without a dilution solvent, promotes rapid reaction (e.g. 30 minutes) among components of the second mixture, suppresses hydrolysis, branching and other side reactions, and facilitates brine separation and washing to obtain a high quality (e.g. an epoxide functionality that approaches maximum theoretical epoxide content with minimal unreacted phenolic groups (e.g. < 6×10⁻⁵ gram mole (gmol) equivalents phenolic OH per gram of resin), low hydroyzable chloride content (e.g. < 15×10⁻⁵ gram mole (gmol) equivalents hydrolyzable chloride per gram of resin), and low ionic chloride content (e.g. < 50ppm) solid epoxy resin that has a low ionic content (e.g. < 50 ppm). The improved process of this invention also allows production of a wider variety of SERs with improved product properties and lower product variability than that which results from a process that is identical to the improved process save for lacking an organic solvent, specifically an alcohol ether solvent. The alcohol ether solvent may be readily removed from the resin mixture by conventional means such as evaporation and readily recovered from the byproduct brine and wash water by conventional means such as distillation.

The mole-equivalent ratio of moles (one mole-equivalent) of epihalohydrin to hydroxyl moieties of the aromatic hydroxyl-containing compound preferably falls within a range of from 0.5:1 to 1:1.

The epihalohydrin and alkali metal hydroxide or alkaline earth metal hydroxide are present in a molar ratio of hydroxide to epihalohydrin that preferably falls within a range of from 0.2:1 to 2:1. The range is more preferably from 1:1 to 1.5:1 1 and still more preferably from 1.01:1 to 1.3:1.

The first mixture may be at any temperature within a range of from 0°C to 150°C prior to adding either the epihalohydrin or the hydroxide (i.e. an alkali metal hydroxide or an alkaline earth metal hydroxide), whichever is appropriate, to the first mixture to form the second mixture. The range is preferably from 20°C to 100°C, and still more preferably from 30°C to 80°C.

The improved process noted above can, in any of its variations, take place under vacuum, at atmospheric pressure or under applied pressure. The pressure is preferably between 0.1 and 10 bar, more preferably between 0.5 and 5 bar, and most preferably at or near atmospheric pressure.

As components of the second mixture react with one another, they do so exothermically, generating heat. The improved process, or any of its steps, may be conducted with cooling to remove heat, under adiabatic conditions, or with heating addition.

In one preferred embodiment, mix reactants or components of the first mixture or the second mixture at one temperature (e.g. 50°C), and allow heat generated by components that react with one another to raise the temperature of the second mixture to a higher temperature (e.g. 80°C) so that the reaction mixture is rapidly brought to the desired reaction temperature (80°C) without requiring either heat removal or heat input. Skilled artisans recognize that, as between two temperatures such as 50°C and 80°C, components of the second mixture react at a faster rate at the higher temperature. In addition, starting at a lower temperature (e.g. 50°C) and taking advantage of generated reaction heat to reach a higher temperature (e.g. 80°C) simplifies reactor design by eliminating a requirement for heat transfer across reactor walls.

In another preferred embodiment, hold the reactants or components at one temperature during addition of the reactants through formation of the second mixture, and then (a) maintain the temperature of the second mixture at that temperature, or (b) increase the temperature of the second mixture via heat of reaction, optionally with added heat. Maintaining a temperature typically requires heat removal via conventional technology such as use of a cooling medium, or evaporation and separate condensation of a portion of the reactor contents. For example, one may remove heat from a reactor that contains the second mixture by allowing the contents of the reactor to rise to the boiling point of the second mixture, condensing the vapors, and returning the condensed vapors to the reactor.

The aromatic hydroxyl-containing compound preferably contains at least one aromatic hydroxyl group and includes phenols, bisphenols, novolac resins, polyvinyl phenols and corresponding amine compounds as taught by Wang et al. (USP 4,499,255) at column 1 line 65 through column 4, line 59. See also, Shirtum et al. (USP 4,877,857) at column 5, line 8 through column 7, line 65. Other preferred phenolic compounds include those taught by Berman et al. (USP 4,727,119) at column 4, lines 12-43. The aromatic hydroxyl-containing compound is preferably at least one polyhydric phenol selected from a group consisting of bisphenol-A, bisphenol-F, a phenol-formaldehyde novolac, a cresolformaldehyde novolac, a bisphenol-A-formaldehyde novolac, a trisphenol, a biphenol, a diphenol, and hydroquinone. Bisphenol-A represents a particularly preferred aromatic hydroxyl-containing compound.

Suitable epihalohydrins include those taught by Shirtum et al. (USP 4,877,857) at column 7, line 65 through column 8, line 14. The epihalohydrin is preferably selected from a group consisting of epichlorohydrin, epibromohydrin, epiiodohydrin, methylepichlorohydrin, methylepibromohydrin, methylepiiodohydrin and combinations thereof, with epichlorohydrin being particularly preferred.

The hydroxide may be an alkali metal hydroxide (e.g. sodium hydroxide or potassium hydroxide) or an alkaline earth hydroxide (e.g. calcium hydroxide). The hydroxide is preferably an alkali metal hydroxide, more preferably sodium hydroxide.

The reaction solvent is any solvent that contains both an ether functionality and an alcohol functionality, preferably an alcohol ether. The reaction solvent is preferably at least one alcohol ether selected from a group consisting of 1-methoxy-2-ethanol, 1-ethoxy-2-ethanol, 1-butoxy-2-ethanol, 1-methoxy-2-propanol, 1-ethoxy-2-propanol, 1-isobutoxy-2-propanol, 1-phenoxy-2-propanol, 1-methoxy-2-butanol, 3-methoxy-1-butanol, 3-methoxy-3-methylbutanol, ethylene glycol monoisopropyl ether, ethylene glycol monoisobutyl ether, ethylene glycol mono-n-butyl ether, and ethylene glycol mono-tert-butyl ether. The alcohol ether preferably has secondary or tertiary alcohol functionality in order to limit its reactivity with the epoxy resin. In addition, the alcohol ether preferably has either a high enough volatility to facilitate separation from the SER during solvent evaporation or a high enough partitioning coefficient to facilitate extraction from a mixture that contains SER and dilution solvent during wash. If one desires to remove the reaction solvent from a mixture comprising the reaction solvent and via evaporation, the alcohol ether preferably has a boiling point at atmospheric pressures of less than (<) 200°C, more preferably < 150°C. The alcohol ether also preferably has a volatility that is high enough to facilitate removal from brine or water by evaporation, distillation or stripping. The alcohol ether preferably has either a lower boiling point than water, or forms a low-boiling azeotrope with water, so that it can be distilled overhead from the water or brine. The alcohol ether more preferably forms an azeotrope with water that boils at a temperature below the boiling point of water at atmospheric pressure. 1-methoxy-2-propanol represents a particularly preferred alcohol ether. The reaction solvent is preferably present in an amount represented by a weight ratio of reaction solvent to epihalohydrin that falls within a range of from 0.1:1 to 10:1, more preferably from 0.5:1 to 5:1.

The optional dilution solvent may be any solvent that increases solubility of solid epoxy resin in an organic phase. The dilution solvent may be a good solvent for the solid epoxy resin either by itself or in combination with the reaction solvent. The dilution solvent, in combination with the reactive solvent and the epoxy resin, preferably forms a second liquid phase upon contact with water and, more preferably, is substantially free of, still more preferably completely free of, any moiety or functionality that reacts with one or more of the aromatic hydroxyl-containing compound, the epihalohydrin, the alkali metal or alkali earth metal hydroxide, and water under the reaction conditions described herein. The dilution solvent has a boiling point at atmospheric pressure that is preferably < 200°C, more preferably < 150°C. In more preferred embodiments, the dilution solvent forms an azeotrope with water that boils at a temperature below the boiling point of water. Examples of suitable dilution solvents include aromatic hydrocarbons, halogenated hydrocarbons, ketones, ethers, and mixtures thereof. Especially suitable dilution solvents include toluene, xylenes, methyl ethyl ketone, methyl isobutyl ketone, and mixtures thereof.

### Examples

Examples (Ex) of the present invention are designated by Arabic numerals and Comparative Examples (Comp Ex or CEx) are designated by capital alphabetic letters. Unless otherwise stated herein, "room temperature" and "ambient temperature" are nominally 25°C.

In the following examples, measure phenolic hydroxyl content by quantitative ultraviolet absorption analysis based on the well known bathochromic shift of the long wavelength maximum of phenols under alkaline conditions (see, for example, Wexler, A. S., Analytical Chemistry, 35 (12), 1936-1943, 1963). Measure viscosity using calibrated Cannon-Fenske tubes in a constant-temperature bath. Measure epoxy equivalent weight, hydrolysable chloride content and ionic chloride content by well-known titration techniques for epoxy resins.

### Example 1

Add 139.5 grams (g) of water, 58.6 g of 50.1 wt % NaOH and 102.4 g of 1-methoxy-2-propanol (DOWANOL™ PM, The Dow Chemical Company) to reactor or reaction flask that is a 1-liter five-necked, glass, baffled, round-bottom flask equipped with an agitator, a thermocouple, a temperature controller, a heating mantle, a nitrogen purge feed line, a refluxing condenser and a bottom drain valve and then introduce a nitrogen purge before adding 110.0 g of bisphenol-A to contents of the reaction flask to form a first mixture. Heat reactor flask contents (first mixture) to a set point temperature of 60°C with stirring to effect substantial dissolution of the bisphenol-A. Add 55.7 g of epichlorohydrin in a single aliquot to the first mixture to form a second mixture which then begins to react and exotherm or generate heat.

The heat generated by the reaction raises the temperature of the contents (reacting second mixture) of the flask to about 78°C. Once the exotherm is complete, set the temperature controller to maintain the temperature at 78-80°C, which is the reflux temperature of the mixture at atmospheric pressure.

At 20 minutes after the epichlorohydrin addition, add 55.8 g of a mixture of 70% toluene and 30% 1-methoxy-2-propanol to the reaction flask with continued stirring. The reactor contents constitute a two-phase mixture that includes an organic phase and an aqueous phase. At 40 minutes after the epichlorohydrin addition, measure phenolic hydroxyl content of the organic phase by ultraviolet (UV) absorption as described above. The phenolic hydroxyl content is 146ppm.

Add 170.1 g of a mixture of 70% toluene and 30% 1-methoxy-2-propanol to the reaction flask, and then add 60.6 g of a mixture of 25% sodium dihydrogen phosphate in water to neutralize the mixture, before reducing the set point temperature to 70 °C. Stir reaction flask contents to mix them, then halt the agitator and allow the mixture to separate into an organic phase and an aqueous brine phase.

Drain the aqueous brine phase from the reaction flask. The brine phase has a pH of approximately 7.

Water wash the organic phase by adding 159.7 g of water, 27.2 g of 1-methoxy-2-propanol and 26.6 g of a mixture of 25% sodium dihydrogen phosphate in water to the reaction flask, stirring contents of the flask to mix them, halting the agitator, allowing the phases to separate, and draining the aqueous phase from the reaction flask. Water wash the organic phase two more times, but with 154 g water and 27 g 1-methoxy-2-propanol.

Rotovap, at temperature of about 185°C and under a vacuum of as low as five (5) mm Hg, the organic phase to effect removal of solvents and residual water from the organic phase and yield a SER with an epoxy equivalent weight of 793 grams per gram-equivalent (g/g-eq)., a phenolic hydroxyl (OH) content of 470 ppm, an ionic chloride content of less than 1 ppm, and a viscosity of 2740 centistokes (cSt) at 150°C.

### Example 2

Replicate Example 1 with changes. First, change the first mixture to 82.8 g of water, 52.5 g of 50.1 wt% NaOH, 102.5 g of 1-methoxy-2-propanol, and 110.1g of bisphenol-A. Second, stir the first mixture at a set point temperature of 55 °C for 10 minutes. Some of the bisphenol-A and a portion of sodium bisphenate formed by a reaction between bisphenol-A and NaOH remains undissolved. Third, add 55.6g of epichlorohydrin to the first mixture to form the second mixture. Fourth, add 50.0 g, rather than 55.8 g, of the mixture of 70% toluene and 30% 1-methoxy-2-propanol to the reaction flask. Prior to neutralization, the phenolic hydroxyl content of the organic phase is 210ppm. Fifth, effect neutralization with 176.0 g of the mixture of 70% toluene and 30% 1-methoxy-2-propanol and 75.6 g of a mixture of 25% sodium dihydrogen phosphate in water.

The SER has an epoxy equivalent weight of 860 g/g-eq., a phenolic OH content of 350 ppm, a hydrolyzable chloride content of 72 ppm, an ionic chloride content of 3 ppm and a viscosity of 2730 cSt at 150°C.

### Example 3

Replicate Example 2 with changes. First, after adding the epichlorohydrin and allowing the temperature to rise by heat of reaction to 78°C, set the temperature controller to a set point temperature of 78°C. Second, at 16 minutes after the epichlorohydrin addition, add 50 g of a mixture of 70% methyl isobutyl ketone and 30% 1-methoxy-2-propanol to the reaction flask rather than a mixture of 70% toluene and 30% 1-methoxy-2-propanol. Prior to neutralization, the phenolic hydroxyl content of the organic phase is 140 ppm. Third, effect neutralization using 175.8 g of a mixture of 70% methyl isobutyl ketone and 1-methoxy-2-propanol and 70.7 g of a mixture of 25% sodium dihydrogen phosphate in water.

The SER had an epoxy equivalent weight of 725 g/g-eq., a phenolic OH content of 300 ppm, a hydrolyzable chloride content of 250 ppm, an ionic chloride content of less than 1 ppm and a viscosity of 2280 cSt at 150°C.

### Example 4

Using the same apparatus as in Example 1, form a first mixture by adding 55.6 g of epichlorohydrin, 127.8 g of 1-methoxy-2-propanol, and 110.0 g of bisphenol-A. Stir the first mixture at a set point temperature of 55°C until the solids dissolve to form a visually clear solution. Form a second mixture by adding 127.8 g of 19.5 wt% NaOH in water to the first mixture evenly over a period of 15 minutes while maintaining the reaction temperature at 55°C. After the NaOH addition is complete, heat the contents of the reaction flask to a set point temperature of 79°C.

Approximately 15 minutes after completing the NaOH in water addition, add 50.0 g of the mixture of 70% toluene and 30% 1-methoxy-2-propanol to the reaction flask. After an additional 20 minutes, the phenolic hydroxyl content of the organic phase of the two-phase mixture is 72 ppm.

After measuring the phenolic hydroxyl content, add to the reaction flask, 209.5 g of a mixture of 70% toluene and 30% 1-methoxy-2-propanol, and then add 25.4 g of water and 77.0 g of a mixture of 25% sodium dihydrogen phosphate in water to neutralize contents of the reaction vessel before dropping the contents to a set point temperature of 70°C as in Example 1.

The SER, isolated as in Example 1, has an epoxy equivalent weight of 925 g/g-eq. and a phenolic OH content of 200 ppm, and a viscosity of 4880 cSt at 150°C.

The foregoing examples demonstrate that the improved process of this invention produces SERs of high quality (e.g. low unreacted phenolic hydroxyl content and a low residual ionic content) in a short reaction time (e.g. less than 60 minutes).

Variations of the examples in accord with various parameters described herein should yield similar results.

## Claims

1. An improved process for preparing a glycidyl ether, which process comprises subjecting a reaction mixture, which reaction mixture comprises an aromatic hydroxyl-containing compound, an epihalohydrin, water, and an inorganic hydroxide that is at least one of alkali metal hydroxide or an alkaline earth metal hydroxide, to conditions sufficient to produce a glycidyl ether resin that is a solid at room temperature, the aromatic hydroxyl-containing compound and the epihalohydrin being present in a mole-equivalent ratio of moles (one mole-equivalent) epihalohydrin to hydroxyl moieties of the aromatic hydroxyl-containing compound that falls within a range of from 0.5:1 to 1:1, and the epihalohydrin and alkali metal hydroxide or alkaline earth metal hydroxide being present in a molar ratio of the hydroxide to the epihalohydrin that falls within a range of from 0.2:1 to 2:1, wherein the improvement comprises adding a reaction solvent to the reaction mixture, the reaction solvent being at least one alcohol ether that comprises both an ether moiety and an alcohol moiety.

2. The process of Claim 1, wherein the solvent is present in a weight ratio of reaction solvent to epihalohydrin that falls within a range of from 0.1:1 to 10:1.

3. The process of Claim 1 or Claim 2, wherein the at least one alcohol ether is selected from a group consisting of 1-methoxy-2-ethanol, 1-ethoxy-2-ethanol, 1-butoxy-2-ethanol, 1-methoxy-2-propanol, 1-ethoxy-2-propanol, 1-isobutoxy-2-propanol, 1-phenoxy-2-propanol, 1-methoxy-2-butanol, 3-methoxy-1-butanol, 3-methoxy-3-methylbutanol, ethylene glycol monoisopropyl ether, ethylene glycol monoisobutyl ether, ethylene glycol mono-n-butyl ether, and ethylene glycol mono-tert-butyl ether.

4. The process of Claim 1, Claim 2 or Claim 3, wherein the reaction solvent has a boiling point at atmospheric pressure that is less than 200 degrees centigrade.

5. The process of any of Claims 1 through 4, wherein the reaction solvent forms an azeotrope with water, which azeotrope boils at a temperature less than a temperature at which water boils at atmospheric pressure.

6. The process of any of Claims 1 through 5, wherein the solvent also comprises an amount of a dilution solvent, the dilution solvent being substantially free of any moiety that reacts with one or more of the aromatic hydroxyl-containing compound, the epihalohydrin, the alkali metal hydroxide or alkaline earth metal hydroxide, and water.

7. The process of any of Claims 1 through 6, wherein the inorganic hydroxide is at least one alkali metal hydroxide or alkaline earth metal hydroxide selected from a group consisting of sodium hydroxide, potassium hydroxide, and calcium hydroxide; the aromatic hydroxyl-containing compound is at least one polyhydric phenol selected from a group consisting of bisphenol-A, bisphenol-F, a phenol-formaldehyde novolac, a cresolformaldehyde novolac, a bisphenol-A-formaldehyde novolac, a trisphenol, a biphenol, a diphenol, and hydroquinone; and the epihalohydrin is at least one member of a group consisting of epichlorohydrin, epibromohydrin, epiiodohydrin, methylepichlorohydrin, methylepibromohydrin, and methylepiiodohydrin.

8. The process of any of Claims 1 through 7, wherein the conditions comprise a temperature within a range of from 0 degrees centigrade to 150 degrees centigrade, and a pressure within a range of from 0.1 bar to 10 bar.

9. The process of Claim 1, wherein the aromatic hydroxyl-containing compound, said compound having an initial content of unreacted phenoloxyl groups, the inorganic hydroxide, the water and the reaction solvent form a first mixture to which the epihalohydrin is added to form a second mixture.

10. The process of Claim 1, wherein the aromatic hydroxyl-containing compound, said compound having an initial content of unreacted phenoloxyl groups, the epihalohydrin and the reaction solvent form a first mixture to which the inorganic hydroxide is added to form a second mixture.

## Patentansprüche

1. Ein verbessertes Verfahren zum Zubereiten eines Glycidylethers, wobei das Verfahren beinhaltet, dass eine Reaktionsmischung, die eine aromatisches Hydroxyl enthaltende Verbindung, ein Epihalogenhydrin, Wasser und ein anorganisches Hydroxid, das mindestens eines von Alkalimetallhydroxid oder einem Erdalkalimetallhydroxid ist, beinhaltet, Bedingungen ausgesetzt wird, die ausreichend sind, um ein Glycidyletherharz, das bei Raumtemperatur ein Feststoff ist, herzustellen, wobei die aromatisches Hydroxyl enthaltende Verbindung und das Epihalogenhydrin in einem Moläquivalent-Verhältnis von Molen (ein Moläquivalent) Epihalogenhydrin zu Hydroxyl-Resten der aromatisches Hydroxyl enthaltenden Verbindung, das in einen Bereich von 0,5:1 bis 1:1 fällt, vorhanden sind und das Epihalogenhydrin und das Alkalimetallhydroxid oder Erdalkalimetallhydroxid in einem Molverhältnis des Hydroxids zu dem Epihalogenhydrin, das in einen Bereich von 0,2:1 bis 2:1 fällt, vorhanden sind, wobei die Verbesserung das Hinzufügen eines Reaktionslösungsmittels zu der Reaktionsmischung beinhaltet, wobei das Reaktionslösungsmittel mindestens ein Alkoholether ist, der sowohl einen Etherrest als auch einen Alkoholrest beinhaltet.

2. Verfahren gemäß Anspruch 1, wobei das Lösungsmittel in einem Gewichtsverhältnis von Reaktionslösungsmittel zu Epihalogenhydrin, das in einen Bereich von 0,1:1 bis 10:1 fällt, vorhanden ist.

3. Verfahren gemäß Anspruch 1 oder Anspruch 2, wobei der mindestens eine Alkoholether ausgewählt ist aus einer Gruppe, die aus Folgendem besteht: 1-Methoxy-2-ethanol, 1-Ethoxy-2-ethanol, 1-Butoxy-2-ethanol, 1-Methoxy-2-propanol, 1-Ethoxy-2-propanol, 1-Isobutoxy-2-propanol, 1-Phenoxy-2-propanol, 1-Methoxy-2-butanol, 3-Methoxy-1-butanol, 3-Methoxy-3-methylbutanol, Ethylenglykolmonoisopropylether, Ethylenglykolmonoisobutylether, Ethylenglykolmono-n-butylether und Ethylenglykolmono-tert-butylether.

4. Verfahren gemäß Anspruch 1, Anspruch 2 oder Anspruch 3, wobei das Reaktionslösungsmittel bei atmosphärischem Druck einen Siedepunkt aufweist, der niedriger als 200 Grad Celsius ist.

5. Verfahren gemäß einem der Ansprüche 1 bis 4, wobei das Reaktionslösungsmittel mit Wasser ein Azeotrop bildet, wobei das Azeotrop bei einer Temperatur siedet, die niedriger als eine Temperatur ist, bei der Wasser bei atmosphärischem Druck siedet.

6. Verfahren gemäß einem der Ansprüche 1 bis 5, wobei das Lösungsmittel auch eine Menge eines Verdünnungslösungsmittel beinhaltet, wobei das Verdünnungslösungsmittel im Wesentlichen frei von jeglichem Rest ist, der mit einem oder mehreren von der aromatisches Hydroxyl enthaltenden Verbindung, dem Epihalogenhydrin, dem Alkalimetallhydroxid oder Erdalkalimetallhydroxid und Wasser reagiert.

7. Verfahren gemäß einem der Ansprüche 1 bis 6, wobei das anorganische Hydroxid mindestens ein Alkalimetallhydroxid oder Erdalkalimetallhydroxid ist, das aus einer Gruppe ausgewählt ist, die aus Natriumhydroxid, Kaliumhydroxid und Calciumhydroxid besteht; wobei die aromatisches Hydroxyl enthaltende Verbindung mindestens ein mehrwertiges Phenol ist, ausgewählt aus einer Gruppe, die aus Folgendem besteht:
Bisphenol-A, Bisphenol-F, einem Phenol-Formaldehyd-Novolak, einem Kresol-Formaldehyd-Novolak, einem Bisphenol-A-Formaldehyd-Novolak, einem Trisphenol, einem Biphenol, einem Diphenol und Hydrochinon; und wobei das Epihalogenhydrin mindestens ein Mitglied einer Gruppe ist, die aus Epichlorhydrin, Epibromhydrin, Epiiodhydrin, Methylepichlorhydrin, Methylepibromhydrin und Methylepiiodhydrin besteht.

8. Verfahren gemäß einem der Ansprüche 1 bis 7, wobei die Bedingungen eine Temperatur in einem Bereich von 0 Grad Celsius bis 150 Grad Celsius und einen Druck in einem Bereich von 0,1 Bar bis 10 Bar beinhalten.

9. Verfahren gemäß Anspruch 1, wobei die aromatisches Hydroxyl enthaltende Verbindung, die einen Anfangsgehalt von unreagierten Phenoloxylgruppen aufweist, das anorganische Hydroxid, das Wasser und das Reaktionslösungsmittel bilden eine erste Mischung, zu der das Epihalogenhydrin hinzugefügt wird, um eine zweite Mischung zu bilden.

10. Verfahren gemäß Anspruch 1, wobei die aromatisches Hydroxyl enthaltende Verbindung, die einen Anfangsgehalt von unreagierten Phenoloxylgruppen aufweist, das Epihalogenhydrin und das Reaktionslösungsmittel bilden eine erste Mischung, zu der das anorganische Hydroxid hinzugefügt wird, um eine zweite Mischung zu bilden.

## Revendications

1. Un procédé amélioré pour préparer un éther glycidylique, lequel procédé comprend soumettre un mélange réactionnel, lequel mélange réactionnel comprend un composé contenant de l'hydroxyle aromatique, une épihalohydrine, de l'eau, et un hydroxyde inorganique qui est au moins soit un hydroxyde de métal alcalin, soit un hydroxyde de métal alcalino-terreux, à des conditions suffisantes pour produire une résine d'éther glycidylique qui est un solide à température ambiante, le composé contenant de l'hydroxyle aromatique et l'épihalohydrine étant présents dans un rapport équivalent molaire entre les moles (un équivalent molaire) d'épihalohydrine et les parties hydroxyle du composé contenant de l'hydroxyle aromatique qui tombe dans une plage allant de 0,5/1 à 1/1, et l'épihalohydrine et l'hydroxyde de métal alcalin ou l'hydroxyde de métal alcalino-terreux étant présents dans un rapport molaire entre l'hydroxyde et l'épihalohydrine qui tombe dans une plage allant de 0,2/1 à 2/1, dans lequel l'amélioration comprend l'ajout d'un solvant de réaction au mélange réactionnel, le solvant de réaction étant au moins un alcool-éther qui comprend à la fois une partie éther et une partie alcool.

2. Le procédé de la revendication 1, dans lequel le solvant est présent dans un rapport pondéral entre le solvant de réaction et l'épihalohydrine qui tombe dans une plage allant de 0,1/1 à 10/1.

3. Le procédé de la revendication 1 ou de la revendication 2, dans lequel cet au moins un alcool-éther est sélectionné dans un groupe consistant en le 1-méthoxy-2-éthanol, le 1-éthoxy-2-éthanol, le 1-butoxy-2-éthanol, le 1-méthoxy-2-propanol, le 1-éthoxy-2-propanol, le 1-isobutoxy-2-propanol, le 1-phénoxy-2-propanol, le 1-méthoxy-2-butanol, le 3-méthoxy-1-butanol, le 3-méthoxy-3-méthylbutanol, l'éther monoisopropylique d'éthylène glycol, l'éther monoisobutylique d'éthylène glycol, l'éther mono-n-butylique d'éthylène glycol, et l'éther mono-tert-butylique d'éthylène glycol.

4. Le procédé de la revendication 1, de la revendication 2 ou de la revendication 3, dans lequel le solvant de réaction a un point d'ébullition à la pression atmosphérique qui est inférieur à 200 degrés centigrades.

5. Le procédé de n'importe lesquelles des revendications 1 à 4, dans lequel le solvant de réaction forme un azéotrope avec l'eau, lequel azéotrope bout à une température inférieure à une température à laquelle l'eau bout à la pression atmosphérique.

6. Le procédé de n'importe lesquelles des revendications 1 à 5, dans lequel le solvant comprend aussi une quantité d'un solvant de dilution, le solvant de dilution étant substantiellement dépourvu de toute partie réagissant avec un ou plusieurs éléments parmi le composé contenant de l'hydroxyle aromatique, l'épihalohydrine, l'hydroxyde de métal alcalin ou l'hydroxyde de métal alcalino-terreux, et l'eau.

7. Le procédé de n'importe lesquelles des revendications 1 à 6, dans lequel l'hydroxyde inorganique est au moins un hydroxyde de métal alcalin ou un hydroxyde de métal alcalino-terreux sélectionné dans un groupe consistant en l'hydroxyde de sodium, l'hydroxyde de potassium, et l'hydroxyde de calcium ; le composé contenant de l'hydroxyle aromatique est au moins un phénol polyhydrique sélectionné dans un groupe consistant en le bisphénol-A, le bisphénol-F, une novolaque phénolformaldéhyde, une novolaque crésol-formaldéhyde, une novolaque bisphénol-A-formaldéhyde, un trisphénol, un biphénol, un diphénol, et l'hydroquinone ; et l'épihalohydrine est au moins un élément d'un groupe consistant en l'épichlorhydrine, l'épibromhydrine, l'épiiodhydrine, la méthylépichlorhydrine, la méthylépibromhydrine, et la méthylépiiodhydrine.

8. Le procédé de n'importe lesquelles des revendications 1 à 7, dans lequel les conditions comprennent une température comprise dans une plage allant de 0 degré centigrade à 150 degrés centigrades, et une pression comprise dans une plage allant de 0,1 bar à 10 bar.

9. Le procédé de la revendication 1, dans lequel le composé contenant de l'hydroxyle aromatique, ledit composé ayant une teneur initiale en groupes phénoloxyles n'ayant pas réagi, l'hydroxyde inorganique, l'eau et le solvant de réaction forment un premier mélange auquel l'épihalohydrine est ajoutée pour former un deuxième mélange.

10. Le procédé de la revendication 1, dans lequel le composé contenant de l'hydroxyle aromatique, ledit composé ayant une teneur initiale en groupes phénoloxyles n'ayant pas réagi, l'épihalohydrine et le solvant de réaction forment un premier mélange auquel l'hydroxyde inorganique est ajouté pour former un deuxième mélange.
